# EUROPEAN PATENT APPLICATION

(11) **EP 3 446 861 A1**
(43) Date of publication of application: **27.02.2019**
(21) Application number: 17186991.0
(22) Date of filing: 21.08.2017
(51) Int. Cl.: B29C 65/68, B29C 65/56, B29C 65/10

(54) **METHOD FOR SEALING MEDICAL DEVICES**

(71) Applicant: Gambro Lundia AB, 220 10 Lund (SE)
(72) Inventor: Assmann, Claudia, 72414 Rangendingen (DE); Flieg, Ralf, 72414 Rangendingen (DE); Freudemann, Wolfgang, 72379 Hechingen (DE); Knoer, Torsten, 72393 Burladingen (DE)
(74) Representative: Perchenek, Nils

(57) **Abstract**

The present disclosure relates to a process for sealing a port of a medical device, comprising closing the opening of the port with a cap or a plug; covering at least the juncture of the port and the plug or cap with shrink-tubing; and shrinking the shrink-tubing to seal the port.

## Description

### Technical Field

The present disclosure relates to a method of sealing ports of medical devices, e.g., filtration and/or diffusion devices like ultrafilters and capillary dialyzers.

### Background of the Invention

During the production of some medical devices, for instance, filtration and/or diffusion devices, ports of the device may have to be permanently sealed. For instance, ports which are used for introducing a liquid or a solid into the device during assembly may have to be permanently sealed to prevent the contents from leaking from the device. Also ports which are redundant in the finished device need to be permanently sealed to prevent escape of any fluid or solid through the respective ports as well as any contamination of the interior space of the device.

Such ports are customarily sealed by welding techniques like friction welding, ultrasound welding or mirror welding, or by pasting a stopper into the port using an adhesive. However, in some cases these sealing techniques cannot be used, because they might impair the product or do not effectively seal the port. Particularly in the case of devices comprising liquids or having fragile structures in their interior, the application of ultrasound or excessive heat might damage the contents of the device. On the other hand, an adhesive might contaminate the contents of the device or fail to provide a permanent seal because of interaction with the contents of the device.

It would be desirable to have an alternative sealing process which reliably provides a permanent seal for a port of a medical device; does not impair the contents of the device; and can be used to seal ports of devices comprising liquids or solids.

### Summary

The present disclosure provides a process for permanently sealing a port of a medical device. The process uses shrink-tubing to seal the juncture of the port and a plug or cap closing the opening of the port.

### Detailed Description

The present disclosure provides a process for sealing a port of a medical device. The process comprises closing the opening of the port with a cap or a plug; covering at least the juncture of the port and the plug or cap with shrink-tubing; and shrinking the shrink-tubing to seal the port.

In one embodiment, the medical device is a filtration and/or diffusion device. In another embodiment, the medical device is a container, in particular a bag or pouch, for liquids, e.g., solutions, dispersions, or gels, and/or particulate material, for instance, powder or granulates. In one embodiment, the medical device is a dialyzer, an ultrafilter, or a plasma filter. In one embodiment, the medical device comprises hollow fiber membranes. In another embodiment, the medical device comprises at least one flat sheet membrane. In a further embodiment, the medical device comprises a liquid. In another embodiment, the medical device comprises particulate material, e.g., polymer beads or carbon particles. In a particular embodiment, the medical device is a filtration and/or diffusion device comprising hollow fiber membranes and being filled with a liquid. In another particular embodiment, the medical device is a filtration and/or diffusion device comprising hollow fiber membranes, particulate material, and a liquid. In one embodiment, the particulate material is located in the space surrounding the hollow fiber membranes.

In one embodiment, the port is comprised of glass, metal, or ceramic. In another embodiment, the port is comprised of a polymer material. Examples include polycarbonate (PC), polypropylene (PP), polymethylmethacrylate (PMMA), and cycloolefin copolymers (COC).

In one embodiment, the port is an integral part of the housing of a filtration and/or diffusion device. In one embodiment, the housing is comprised of a polymer material. Suitable polymer materials include polycarbonate (PC), polypropylene (PP), polymethylmethacrylate (PMMA), and cycloolefin copolymers (COC). In one embodiment, the port is tubular. In one embodiment, the port is a connector of a filtration and/or diffusion device for hemofiltration, hemodiafiltration, or hemodialysis, as described in DIN EN ISO 8637 (2014). In a particular embodiment, the port is a Luer connector. In another particular embodiment, the port is a Hansen connector.

In the first step of the process of the present disclosure, the opening of a port of the medical device to be sealed is closed with a cap or a plug. In one embodiment, the opening of the port is closed by putting a cap over the opening of the port. The cap extends over the orifice of the port and a part of the exterior of the port. The inner diameter of the cap matches the outer diameter of the port, so that the gap between the exterior of the port and the inner wall of the cap is small, e.g., 0.05 to less than 1 mm. In a further embodiment, the outside of the port features a screw thread, and the cap features a matching thread on its inside and is screwed onto the port. In another embodiment, the opening of the port is closed by inserting a plug or stopper into the opening of the port. In one embodiment, the plug has a conical shape, its minimum diameter being smaller than the diameter of the opening of the port and its maximum diameter being larger than the diameter of the opening of the port. After insertion, part of the plug protrudes from the opening of the port. In another embodiment, the plug features a section of cylindrical or conical shape, the maximum diameter of this section being smaller than the inner diameter of the port; and a head section having a diameter matching the outer diameter of the port. After insertion, the head section of the plug covers the opening and the rim of the port. The gap between the rim of the port and the head section of the plug preferably is small, e.g., 0.05 to less than 1 mm.

In one embodiment, the plug is comprised of glass. In another embodiment, the plug is comprised of a polymer material. Examples include polycarbonate (PC), polypropylene (PP), polymethylmethacrylate (PMMA), and cycloolefin copolymers (COC).

In one embodiment, the port to be closed and the plug or cap are comprised of the same material. In another embodiment, the port to be closed and the plug or cap are comprised of materials having similar coefficients of thermal expansion. The use of the same material or of materials having similar thermal expansion coefficients for both the port and the plug or cap prevents the formation of stress cracks during and after sealing of the port.

After the opening of the port has been closed with a plug or cap, at least the juncture of the port and the plug or cap, respectively, is covered with shrink-tubing. The inner diameter of the shrink-tubing is slightly larger than the outer diameter of the port and the outer diameter of the plug or cap, respectively. In one embodiment, the inner diameter of the shrink-tubing is 1 to 10 mm larger than the outer diameter of the port. The shrink-tubing covers at least the juncture of the port and the plug or cap, respectively. In one embodiment, a section of shrink-tubing having a length in the range of from 5 to 30 mm is used to cover the juncture and the adjacent parts of the port and the plug or cap. In another embodiment, a shrink cap, i.e. a shrink-tubing closed at one end, is used to cover parts of the port and the plug or cap, extending over the juncture. In one embodiment, the shrink cap spreads over the juncture and extends beyond it, e.g., by a length in the range of from 5 to 30 mm. In one embodiment, the shrink tubing has a wall strength after shrinking of 0.2 to 2.5 mm.

In one embodiment of the process of the present disclosure, the shrink-tubing is comprised of at least one elastomer, e.g., Viton^{®}. In one embodiment, the shrink-tubing is comprised of at least one polyolefin, silicone, polyvinyl chloride (PVC), or fluoropolymer. Examples of suitable fluoropolymers include polyvinylidene fluoride (PVDF), polytetrafluoroethylene (PTFE), fluorinated ethylene propylene (FEP), and perfluoroalkoxy alkanes (PFA).

In one embodiment, the lumen of the shrink-tubing is coated with an adhesive. Examples of suitable adhesives include polyamide hot melt adhesives, polyolefin hot melt adhesives, fluorinated ethylene propylene (FEP), and perfluoroalkoxy alkanes (PFA). During the shrinking step, the adhesive forms an additional bonding between the tubing and the port, securing the tubing and preventing it from slipping off the port. This is particularly useful with tapered ports.

The shrink-tubing subsequently is shrunk to seal the port. In one embodiment, the shrinking process is effected by heating the shrink-tubing. Depending on the type of shrink-tubing used, the shrink temperature is in the range of from 80 to 330°C, for instance, from 90 to 175°C or from 90 to 120°C.

The shrinking step can be performed prior to or during sterilization of the medical device. In one embodiment of the process, the shrinking of the shrink-tubing is performed by sterilizing the medical device with steam. During steam sterilization, the medical device is heated to a temperature in the range of from 115 to 121°C.

The shrink ratio of the shrink tubing, i.e., the ratio of the diameter prior to the shrinking step to the diameter after the shrinking step, generally is in the range of from 1.3:1 to 5:1, in particular in the range of from 2:1 to 4:1, or from 3:1 to 4:1.

The present disclosure also is directed to the use of shrink-tubing for sealing a port of a medical device. In one embodiment, the lumen of the shrink-tubing is coated with an adhesive.

### Examples

Unless mentioned otherwise, in each of the examples a dialysate port of a hemodialyzer housing comprised of polycarbonate was closed with a plug comprised of polycarbonate. The diameter of the head of the plug was about 15 mm, so that after insertion into the port, its outline was flush with the outer diameter of the dialysate port.

### Example 1

A 30 mm piece of flexible, radiation cross-linked polyolefin tubing coated with polyamide hot melt adhesive on the inside and having an inner diameter of 16 mm (FITCOTUBE^{®} FT800E-HT-16/4; GREMCO GmbH, D-86165 Augsburg) was slid over the plug and the port, so that the end of the tubing extended 5 mm beyond the head of the plug. The material has a shrink temperature of 110°C, and a shrink ratio of 4:1.

The device was steam-sterilized at 116°C in an autoclave for 2 hours. Total time in the autoclave was 4 hours. A leak test performed on the device confirmed that the seal was tight at a pressure of 1.6 bar in the device.

### Example 2

a) A 30 mm piece of flexible, radiation cross-linked tubing coated with polyamide-based hot melt adhesive on the inside and having an inner diameter of 19 mm (FITCOTUBE^{®} FT881-1900; GREMCO GmbH, D-86165 Augsburg) was slid over the plug and the port, so that the end of the tubing extended 5 mm beyond the head of the plug. The material has a shrink temperature of 120°C, and a shrink ratio of 2:1.
   Several devices were heated to 120°C in an oven and kept at 120°C for 2 hours. A leak test was performed on the devices to determine whether or not the seal was tight at a pressure of 1.6 bar in the device. The devices were subsequently sterilized with steam at 116°C for 2 hours and the leak test was repeated. The second leak test confirmed that the seal of those devices that had passed the first leak test was still intact. Furthermore, even those devices that had not passed the first leak test had been sealed by the steam sterilization process and passed the second leak test.
b) A 30 mm piece of flexible, radiation cross-linked tubing coated with polyamide-based hot melt adhesive on the inside and having an inner diameter of 19 mm (FITCOTUBE^{®} FT888-19/6 transparent; GREMCO GmbH, D-86165 Augsburg) was slid over the plug and the port, so that the end of the tubing extended 5 mm beyond the head of the plug. The material has a shrink temperature of 120°C, and a shrink ratio of 3:1.

The device was heated to 120°C in an oven and kept at 120°C for 2 hours. A leak test performed on the device confirmed that the seal was tight at a pressure of 1.6 bar in the device. The device was subsequently sterilized with steam at 121°C for 21 minutes and the leak test was repeated. The leak test confirmed that the seal was still intact.

### Example 3

In this example, a screw-on polypropylene cap having an outer diameter of 18.4 mm was used to close the dialysate port instead of a polycarbonate plug.

A 30 mm piece of flexible, radiation cross-linked tubing coated with polyamide-based hot melt adhesive on the inside and having an inner diameter of 25.4 mm (FITCOTUBE^{®} FT881-2540; GREMCO GmbH, D-86165 Augsburg) was slid over the cap and the port, so that the end of the tubing extended 5 mm beyond the head of the cap. The material has a shrink temperature of 120°C, and a shrink ratio of 2:1.

The device was heated to 120°C in an oven for 2 hours. A leak test performed on the device confirmed that the seal was tight at a pressure of 1.6 bar in the device. The device was subsequently sterilized with steam at 116°C in an autoclave for 2 hours and the leak test was repeated. The leak test confirmed that the seal still was intact.

## Claims

1. A process for sealing a port of a medical device, comprising closing the opening of the port with a cap or a plug; covering at least the juncture of the port and the plug or cap with shrink-tubing; and shrinking the shrink-tubing to seal the port.

2. The process of claim 1, wherein the medical device is a filtration and/or diffusion device.

3. The process of claim 2, wherein the filtration and/or diffusion device is a dialyzer.

4. The process of claim 1, wherein the medical device is a bag or pouch.

5. The process of any one of claims 1 to 4, wherein the medical device comprises a liquid.

6. The process of any one of claims 1 to 5, wherein the medical device comprises particulate material.

7. The process of any one of claims 1 to 6, wherein the lumen of the shrink-tubing is coated with an adhesive.

8. The process of any one of claims 1 to 7, wherein the shrinking of the shrink-tubing is performed by sterilizing the medical device with steam.

9. Use of shrink-tubing for sealing a port of a medical device.

10. The use of claim 9, wherein the lumen of the shrink-tubing is coated with an adhesive.

11. The use of claims 9 or 10, wherein the medical device is a filtration and/or diffusion device comprising hollow fiber membranes and being filled with a liquid.

12. The use of claim 11, wherein the medical device comprises particulate material located in the space surrounding the hollow fiber membranes.
